# EUROPEAN PATENT APPLICATION

(11) **EP 4 023 256 A2**
(43) Date of publication of application: **06.07.2022**
(21) Application number: 21383145.6
(22) Date of filing: 15.12.2021
(51) Int. Cl.: A61L 9/20, C02F 1/32, F24F 8/22

(54) **DISINFECTION DEVICE FOR FLUIDS CIRCULATING THROUGH DUCTS BY MEANS OF UV RADIATION**

(30) Priority: 04.01.2021 ES 202130001
(71) Applicant: Luminalia Ingenieria y Fabricación, S.L, 33199 Asturias (ES); Universidad Complutense De Madrid, 28040 Madrid (ES)
(72) Inventor: Vázquez Moliní, Daniel, Madrid (ES); Alvarez Fernandez-Balbuena, Antonio, Madrid (ES); Alda Serrano, Javier, Madrid (ES); Martinez Antón, Juan Carlos, Madrid (ES); Estrada Vena, Luis, Asturias (ES); Prada Pérez, Luis, Asturias (ES)
(74) Representative: Álvarez López, Sonia

(57) **Abstract**

A disinfection device (1) for fluids circulating through ducts (2) by means of UV radiation, comprising:
-a chamber (3) provided with means for the attachment thereof to the duct (2) to which the device is applied, and being open towards said duct (2),
-whose chamber (3) interiorly comprises at least one elliptical reflector (4),
- comprising at least one UVC ultraviolet light source (5) disposed at the first focus (41) of the reflector (4), and
-where the reflector (4) is disposed in the circulation zone (6) of the flow.

## Description

### OBJECT OF THE INVENTION

The present invention relates to a disinfection device for fluids circulating through ducts, which disinfects said fluids by means of ultraviolet radiation, and is usable in the technical sector of viral pathogen disinfection, in all types of ducting installations through which a liquid or gas circulates.

### BACKGROUND OF THE INVENTION

The quality of the air in enclosed premises depends on the natural or artificial ventilation capacity of the same. In the case of enclosures with air handling and/or forced ventilation, the air is directed - to control its temperature - towards air handling systems, which can operate - or not - by means of air recirculation techniques. In any case, the passage of these gaseous fluids through the ventilation or air handling systems can be exploited to improve their quality, as is achieved with various mechanical methods of trapping particles, such as filters for dust, pollen or allergens.

The pandemic caused by the coronavirus SARS-CoV-2 has revealed airborne transmission as a possible source of contagion, and therefore, as a possible area for improving air quality in enclosed premises. Therefore, the incorporation of disinfection elements in the air handling or ventilation units, or as elements used specifically for this purpose, becomes a valid strategy for the improvement of air quality in terms of the reduction and eventual elimination of its pathogenic load. These same types of disinfection systems can be adapted to fluids, for example water, in situations where the control of other types of pathogens, such as Legionella-causing bacteria, may be of interest. In this sense, we can exemplify the germicidal capabilities of UV radiation by using the COVID-19 pathogen.

Coronaviruses are members of the group Coronaviridae, they have a DNA surrounded by a corona-like helical envelope (Ryan 1994, Ryan, K. J. 1994). Sherris Medical Microbiology. Appleton & Lange, Norwalk.). Coronavirus SAR studies indicate a very high degree of infection (He, Y., Jiang, Y., Xing, Y. B., Zhong, G. L., Wang, L., Sun, Z. J., Jia, H., Chang, Q., Wang, Y., Ni, B., and Chen, S. P. (2003). "Preliminary result on the nosocomial infection of severe acute respiratory syndrome", so the disinfection of spaces is necessary to contain the spread. There are several studies that indicate that the infection capability of some types of coronavirus is reduced by means of UV light. For example (Darnell ME, Inactivation of the coronavirus that induces severe acute respiratory syndrome, SARS-CoV. J Virol Methods. 2004 Oct;121(1):85-91.) indicate that the SARS-CoV Coronavirus was inactivated using ultraviolet (UV) light at the wavelength of 254nm for a certain period of time.

Ultraviolet light is an energy of between 100 and 400nm (ICD 155:2003). UVA comprises 315 to 400nm, UVB 280 to 315nm and UVC 100 to 280nm. This standard defines the term UVGI as ultraviolet light with germicidal irradiation. This germicidal action follows the main laws, such as the law of the inverse of the square of distance, or the Bunsen-Roscoe Law of reciprocity, which states that the exposure time is inversely proportional to the irradiation of the sample for the same level of inactivation.

There are various disinfection methods such as HEPA filtering, heat, irradiation. For example, patent WO2007081401A2 "Use of ultraviolet germicidal irradiation in health care environments" discusses a UV disinfection method in conjunction with an ozone generator that operates when no people are present. In this patent and others, there is no automatic UV disinfection system that monitors the room and the time required for disinfection. However, there are articles such as [Bedell2016, Bedell K, Buchaklian AH, Perlman S. "Efficacy of an Automated Multiple Emitter Whole-Room Ultraviolet-C Disinfection System Against Coronaviruses MHV and MERS-CoV". Infect Control Hosp Epidemiol. 2016;37(5):598-9] which mention a UV-C disinfection system for surfaces that inactivates DNA-type viruses such as MHV-A59, MERS-CoV and SARS-CoV.

The patent RO119069B1 INSTALLATION FOR AIR AND SURFACES DISINFECTION USING VARIABLE BEAM OF NON-IONIZING RADIATION refers to a system in which cylindrical elements are used.

Patent US2017232127A1 on DISINFECTION SYSTEM is a system that uses ozone.

Patent WO2007085262A1 SYSTEM FOR REMOVAL OF AIRBORNE CONTAMINANTS relates to a chamber with a lamp that generates ultraviolet radiation and ozone, and prevents the radiation source from getting dirty.

Patent WO9826810A1 METHOD AND DEVICE FOR SHEER FILTERING AND DISINFECTION OF BREATHING AIR is a system comprising a duct in which various elements such as a carbon filter and a UV radiation source are located, but nothing is specified about the type of geometry of the chamber in which the source is located.

Patent WO02079701A1 on DISINFECTION SYSTEM AND METHOD OF USING SAME has a chamber wherein oxygen is converted into ozone which is subsequently directed to a chamber that eliminates it, in such a way as to prevent the risk of contact therewith.

The patent UA84463U DEVICE FOR DISINFECTION OF AIR BY ULTRAVIOLET RADIATION is a system that has a tripod enabling its mounting on the objects to be disinfected.

Patent WO2007026050A1 AIR DISINFECTION DEVICE has a chamber on which no type of requirement is fixed, where there is an ultraviolet light to produce ozone.

Patent WO2019045777A1 AIR TREATMENT SYSTEM AND METHOD has a fan with a 90° elbow wherein LED sources for UV light radiation are disposed. The ratio between the so-called sanitary chamber and the cross-section may be 25.

Patent WO03039604A2 ULTRAVIOLET DISINFECTING APPARATUS has a tube where radiation is propagated by total internal reflection.

Patent WO9709073A1 PHOTOCATALYTIC AIR DISINFECTION is a System employing the upper layer of a filter having a material with a bonding energy that enables a photocatalytic process to be generated.

Patent EP2100624A1 Device and method for disinfecting air, has a chamber with two UV radiation sources, one of which is for generating ozone and the other is different from the first.

These documents present different arrangements and disinfection systems that use UV radiation and ozone, but do not describe any characteristic that amplifies or intensifies the effects of the radiation, so that they require UV elements of a greater presence and cost compared to the device of the invention.

### DESCRIPTION OF THE INVENTION

The disinfection device of the invention for fluids circulating through ducts by means of UV radiation comprises, in its most generic embodiment:
- a chamber having means for its attachment to the duct to which the device is applied, open toward said duct for the flow to circulate therethrough,
- whose chamber comprises interiorly at least one elliptical reflector (which therefore comprises two foci),
- which comprises at least one ultraviolet light source (one or more) of suitable wavelength (UVC light) arranged in a first focus of the reflector, and -where the reflector, and particularly its second focus, is disposed in the flow circulation zone.

In this way, in the area corresponding to the second focus of the reflector, a crossed concentration of all the trajectories of the light generated at the UV light source disposed at the first focus is achieved, increasing the light intensity and the disinfection in this area. But not only that, the reflector thus configured forms a light resonance casing wherein the light circulates and is strengthened throughout the section, and accentuates the disinfectant effects when passing through the second common focus, and also through the first focus - or through all the foci in compound elliptical geometries - so that all the foci will be areas of greater disinfection and will be suitable for disinfecting a circulating flow, and will even generate other areas with an accentuated effect.

Therefore, this is a disinfection device for fluid ducts (air, water, etc.) where said flow circulates interiorly, and which has a reflector of elliptical geometry, which can be simple or compound (materialized by one or more ellipses) and where the UV radiation emitted is confined within the reflector, ensuring that it passes, depending on the coefficient of reflection of the internal surfaces, numerous times through the other areas in a resonant manner, especially through the second focus, thus exerting a greater and better inactivation of the pathogenic elements found in the fluid that circulates through the duct.

The device will be incorporated in a duct, for example an air duct, and thanks to its geometry and materials enables the optimal use of the radiation emitted. The invention is targeted at the elimination of the viral pathogens found in the interior of ducts, for example air handling ducts. Furthermore, it will have PCBs (printed circuit boards) or casings to hold the UV-C sources.

The UVC light generated is directed towards the second focus, the location of the disinfection zone, and can also continue to propagate towards the rest of the geometry and iteratively to return to the disinfection zone. Depending on the inclination of the light path, the successive steps and returns will be carried out at different heights, achieving greater effectiveness. The ducting shall connect with the device either on the plane perpendicular to the ellipses or on the transverse axis thereof, always occupying areas in which the possibility of radiation leakage is minimal.

The reflector shall have a reflectance as high as possible to enable the radiation to be reflected effectively as many times as possible, and may be comprised of one or more elliptical sections wherein a focus of the same is made to coincide, so that the radiation passes from one section to another, but maintaining the same properties of light recirculation. This light resonance casing can be terminated by flat or curved reflective surfaces, depending on the optical characteristics of the light sources.

### BRIEF DESCRIPTION OF THE DRAWINGS

Figure 1.- Portrays the device of the invention in an embodiment where the reflector has simple elliptical geometry and the flow circulation is aligned with the generatrix of the reflector.
Figure 2.- Portrays the device of the invention in the embodiment of Figure 1, attached to a horizontal duct.
Figure 3.- Portrays the device of the invention in an embodiment where the reflector has simple elliptical geometry and the flow circulation is perpendicular to the major axis of the section.
Figure 4.- Portrays the device of the invention in the embodiment of figure 3, attached to a vertical duct.
Figure 5.- Portrays a cross-section of a reflector of simple elliptical geometry and the resonant trajectories -in the transverse plane- of the light generated in the UVC light source, and which are reflected successively and tend to move closer and closer to the horizontal plane that contains the foci of the elliptical section.
Figure 6.- Portrays a cross-section of a reflector formed by an elliptical section composed of two simple elliptical sections aligned by their major axis, where the second focus of both simple sections coincides, and at the first focus of each simple section a light source is disposed, where it may be seen that the successive resonant paths of the UV light generated travel through both sections.

### DESCRIPTION OF A PRACTICAL EMBODIMENT OF THE INVENTION

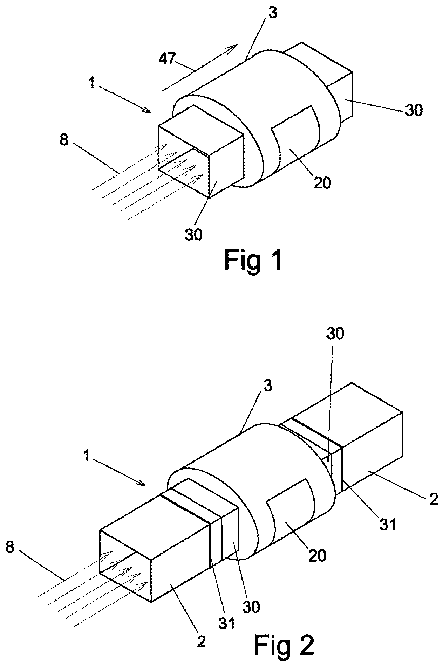

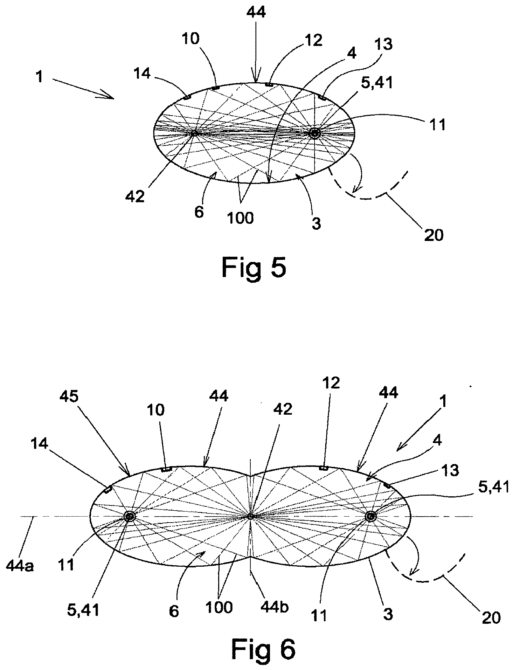

The disinfection device (1) by UV radiation for fluids circulating through ducts (2) of the invention comprises:
- a chamber (3) equipped with means for the attachment thereof to the duct (2) whereto the device is applied, for example collars (30) and flanges (31), for the insertion and tightening thereof against open ends of said ducts (2), incorporating them between the same, as may be seen in Figs. 1 to 4, with their open portion towards said duct (2),
- whose chamber (3) interiorly comprises at least one elliptical reflector (4) (see figs. 5 and 6), comprising two foci (41, 42),
- comprising at least one UVC ultraviolet light source (5) (one or more) disposed at the first focus (41) of the reflector (4), and
- where the reflector (4), and particularly its second focus (42), is disposed in the flow circulation zone (6).

In a first, more simple embodiment (see fig. 5), the reflector (4) comprises a simple elliptical section (44) (in the form of a simple ellipse), at whose first focus (41) the UVC ultraviolet light source (5) is disposed, and at whose the second focus (42) the irradiation to the flow is greatly enhanced, as may be seen in fig. 5.

In a variant of this first embodiment, not depicted, the reflector (4) can comprise a simple elliptical section (44), in each of whose foci (41, 42) is disposed at least one ultraviolet light source (2), generating in the vicinity of each focus a greater concentration of UV light and greater disinfection therein. That is, each focus is a first focus and a second focus at the same time. In either of these two embodiments, it may be seen in fig. 5 how the successively reflected trajectories of the light (100) tend to come closer and closer to the horizontal plane containing the foci (41, 42) of the ellipse, and greater disinfection is achieved in this area, which makes it ideal for using ducts of oblong section.

In another variant of the invention, the reflector (4) may comprise a composite elliptical section (45), formed by a plurality of simple elliptical sections (44) whose second foci (42) are positionally coincident, and at whose first foci (41) an ultraviolet light source (5) (one or several) is disposed. For example, fig. 6 shows that the reflector (4) comprises a composite elliptical section (45), formed by two simple elliptical sections (44) aligned by their major axis (44a), whose second foci (42) are positionally coincident in the line of intersection (44b) of both simple elliptical sections (44), and at whose first foci (41) an ultraviolet light source (5) is disposed. This further enhances the resonant effect as all the trajectories (100) pass through the two simple elliptical sections (44), and particularly through all the foci (41, 42).

Likewise, in any of the variants described, the ultraviolet light source(s) (5) may be disposed slightly offset (at a distance proportional to the diameter of the emitting source, between one and 10 times its diameter, for example) from the first focus (41) where they are installed, to prevent the radiation from passing once more through the same source and overheating the same, although this feature is not portrayed in the figures.

It should be mentioned that, since the reflector must be continuous, the chamber (3) can be integrated by the structure of the reflector (4) itself, as shown in the figures. That is, the chamber is defined by the reflector. They are the same thing except in the collars (30) linking them to the duct (2). This saves material and costs.

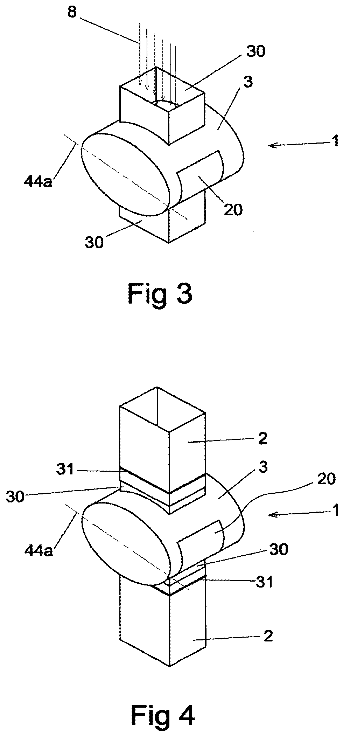

The reflector (4) may be positioned so that the direction of flow (8) is aligned with the direction of the generatrix (47) of the reflector (4) (for this purpose the entrances to the chamber (2) will be at both ends of the chamber, in the direction of said generatrix, as seen in figs. 1 and 2), or alternatively, the reflector (4) may be positioned such that the direction of flow (8) is perpendicular to the major axis (44a) of the section of the reflector (4), as seen in figs. 3 and 4. For this purpose, the entrances to the chamber will be at both ends of the direction of said generatrix.

It should be mentioned that the ultraviolet light sources (5) may be mounted on a printed circuit board or in lamp holders (sockets), not portrayed.

The invention has provided for the possible arrangement of a switch/regulator, not portrayed, for the light intensity of the ultraviolet light sources (5), associated with (see figs. 5 and 6) a measurement means (flow sensors (10)) or the establishment (flow impellers) of the flow rate. This switch/regulator, in cooperation with the flow sensors (10), will also be responsible for switching off the ultraviolet light sources (5) in the event that there is no air circulation.

Likewise, the possible disposition of filters (11) blocking the wavelengths between 100 nm and 220 nm and disposed at the ultraviolet light sources (5) has been provided for, to prevent the generation of ozone, since these wavelengths are those at which ozone is generated when irradiating the air (for ducts where air circulates).

The possible disposition of a CO2 sensor (12) to assess the possible overload of pathogens in the air and the entry into operation of the device with more or less light intensity has also been envisaged.

In addition, the chamber (2) may comprise hatches (20) for maintenance; in this case it may have a hatch-open sensor (13), associated with the switch/regulator or with the power supply of the ultraviolet light sources (5) to cut off said power supply in the event of the opening of the hatch.

Likewise, a UV radiation sensor (14) (associated with a warning panel and/or a control unit) may be disposed in order to verify the correct operation of the ultraviolet light sources (5).

Finally, the preferred geometry of the reflector is defined by tests carried out where, for an air handling duct with a square section of 40 cm wide by 40 cm high, it comprises a simple elliptical reflector (4) with a longitudinal axis of 70 cm, a focal length of 10 cm and a vertical axis of 49 cm, so that it is centred in the air handling duct, and with a length in the direction of the generatrix of 60 cm, with a single UV-C radiation source at the first focus (41) of the section.

Having sufficiently described the nature of the invention, as well as the manner in which it is carried out in practice, it should be noted that the provisions indicated above and shown in the accompanying drawings are subject to modifications in details insofar as they do not alter the essence of the invention.

## Claims

1. A disinfection device (1) for fluids circulating through ducts (2) by means of UV radiation, **characterized in that** it comprises:
- a chamber (3) provided with means for the attachment thereof to the duct (2) to which the device is applied, and being open towards said conduit (2),
- whose chamber (3) interiorly comprises at least one elliptical reflector (4),
- comprising at least one UVC ultraviolet light source (5) disposed at the first focus (41) of the reflector (4), and
- where the reflector (4) is disposed in the circulation zone (6) of the flow.

2. A disinfection device (1) for fluids circulating through ducts (2) by means of UV radiation, as claimed in claim 1, **wherein** the reflector (4) comprises a simple elliptical section (44), in whose first focus (41) the UVC ultraviolet light source (5) is disposed.

3. A disinfection device (1) for fluids circulating through ducts (2) by means of UV radiation, as claimed in claim 2, **wherein** the reflector (4) comprises a simple elliptical section (44), at each of whose foci (41, 42) at least one ultraviolet light source (2) is disposed.

4. A disinfection device (1) for fluids circulating through ducts (2) by means of UV radiation, as claimed in claim 1, **wherein** the reflector (4) comprises a composite elliptical section (45), formed by a plurality of simple elliptical sections (44) whose second foci (42) are positionally coincident, and at whose first foci (41) an ultraviolet light source (5) is disposed.

5. A disinfection device (1) for fluids circulating through ducts (2) by means of UV radiation, as claimed in claim 4, **wherein** the reflector (4) comprises a composite elliptical section (45), formed by two simple elliptical sections (44) aligned by their major axis (44a), whose second foci (42) are positionally coincident in the line of intersection (44b) of both simple elliptical sections (44), and at whose first foci (41) an ultraviolet light source (5) is disposed.

6. A disinfection device (1) for fluids circulating through ducts (2) by means of UV radiation, as claimed in any of the preceding claims, **wherein** some or all of the ultraviolet light sources (5) are disposed slightly offset from the focus where it is installed.

7. A disinfection device (1) for fluids circulating through ducts (2) by means of UV radiation, as claimed in any of the preceding claims, **wherein** the chamber (3) is integrated in the structure of the reflector (4).

8. A disinfection device (1) for fluids circulating through ducts (2) by means of UV radiation, as claimed in any of the preceding claims, **wherein** the reflector (4) is positioned so that the direction of flow (8) is aligned with the direction of the generatrix (47) of the reflector (4).

9. A disinfection device (1) for fluids circulating through ducts (2) by means of UV radiation, as claimed in any one of Claims 1 to 7, **wherein** the reflector (4) is positioned so that the direction of flow (8) is perpendicular to the major axis (44a) of the section of the reflector (4).

10. A disinfection device (1) for fluids circulating through ducts (2) by means of UV radiation, as claimed in any of the preceding claims, **wherein** the ultraviolet light sources (5) are mounted on a printed circuit board or in lamp sockets.

11. A disinfection device (1) for fluids circulating through ducts (2) by means of UV radiation, as claimed in any of the preceding claims, **comprising** a switch/regulator of the intensity of the light from the ultraviolet light sources (5), associated with means for measuring or establishing the flow rate.

12. A disinfection device (1) for fluids circulating through ducts (2) by means of UV radiation, as claimed in any of the preceding claims, **comprising** filters (11) for blocking the wavelengths comprised between 100 nm and 220 nm, disposed at the ultraviolet light sources (5).

13. A disinfection device (1) for fluids circulating through ducts (2) by means of UV radiation, as claimed in any of the preceding claims, **comprising** a CO2 sensor (12).

14. A disinfection device (1) for fluids circulating through ducts (2) by means of UV radiation, as claimed in any of the preceding claims, **wherein** the chamber (2) comprises hatches (20) for maintenance

15. A disinfection device (1) for fluids circulating through ducts (2) by means of UV radiation, as claimed in claim 14, **having** a hatch (20) open sensor (13) associated with the switch/regulator (9) or with the power supply of the ultraviolet light sources (5).

16. A disinfection device (1) for fluids circulating through ducts (2) by means of UV radiation, as claimed in any of the preceding claims, **having** a UV radiation sensor (14).
